(19) Europäisches Patentamt / European Patent Office / Office européen des brevets

(11) **EP 3 531 890 B1**

(12) **EUROPÄISCHE PATENTSCHRIFT**

(45) Veröffentlichungstag und Bekanntmachung des Hinweises auf die Patenterteilung:
**01.04.2020 Patentblatt 2020/14**

(51) Int Cl.:
*A61B 3/032* *(2006.01)*      *A61B 3/00* *(2006.01)*
*A61B 3/028* *(2006.01)*

(21) Anmeldenummer: **17808326.7**

(22) Anmeldetag: **18.10.2017**

(86) Internationale Anmeldenummer:
**PCT/EP2017/076580**

(87) Internationale Veröffentlichungsnummer:
**WO 2018/077690 (03.05.2018 Gazette 2018/18)**

(54) **VORRICHTUNGEN, VERFAHREN UND COMPUTERPROGRAMME ZUR BESTIMMUNG DER REFRAKTION DES AUGES**

DEVICES, METHODS, AND COMPUTER PROGRAMS FOR DETERMINING THE REFRACTION OF THE EYE

DISPOSITIFS, PROCÉDÉS ET PROGRAMMES INFORMATIQUES PERMETTANT DE DÉTERMINER LA RÉFRACTION DE L'OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.10.2016 DE 102016120350**

(43) Veröffentlichungstag der Anmeldung:
**04.09.2019 Patentblatt 2019/36**

(73) Patentinhaber: **Carl Zeiss Vision International GmbH**
**73430 Aalen (DE)**

(72) Erfinder:
• **OHLENDORF, Arne**
**72072 Tübingen (DE)**
• **LEUBE, Alexander**
**72072 Tübingen (DE)**

(74) Vertreter: **Sticht, Andreas**
**Kraus & Weisert**
**Patentanwälte PartGmbB**
**Thomas-Wimmer-Ring 15**
**80539 München (DE)**

(56) Entgegenhaltungen:
**EP-A1- 2 606 815**      **DE-A1- 3 102 450**
**US-A1- 2003 053 027**

EP 3 531 890 B1

**Beschreibung**

Die vorliegende Erfindung betrifft Systeme zur Bestimmung der Refraktion, d.h. Lichtbrechung im Auge, insbesondere zum Bestimmen der sphärozylindrischen Refraktion des Auges.

Ein Auge kann eine Fehlsichtigkeit wie beispielsweise Myopie (Kurzsichtigkeit), Hyperopie (Weitsichtigkeit) oder Astigmatismus (Hornhautverkrümmung) aufweisen. Eine derartige Fehlsichtigkeit wird üblicherweise als sphärozylindrische Refraktion mit drei Parametern (Sphäre, Zylinder und Achse) angegeben, wobei Sphäre und Zylinder die sphärische Brechkraft und die zylindrische Brechkraft angeben und mit der Einheit Dioptrie verknüpft sind und die Achse die Achslage als Winkel angibt. Sphäre, Zylinder und Achse sind in der Norm DIN EN ISO 13666:2013-10 in den Abschnitten 11.2, 12.5 bzw. 12.6 definiert und werden in dieser Anmeldung in diesem Sinne verwendet.

Bei der Refraktionsbestimmung kann zwischen subjektiver Refraktionsbestimmung und objektiver Refraktionsbestimmung unterschieden werden. Verfahren zur subjektiven Refraktionsbestimmung beruhen dabei auf einer (subjektiven) Rückmeldung einer zu untersuchenden Person über ihre visuelle Wahrnehmung. Ein Beispiel ist dabei eine Messung auf Basis von Sehtafeln mit immer kleiner werdender Sehzeichen (z.B. Zahlen oder Buchstaben) oder immer kleiner werdenden Symbolen, wobei die zu untersuchende Person Rückmeldung gibt, welche Zeichen sie erkennen kann. Verfahren und Vorrichtung zur objektiven Refraktionsbestimmung benötigen eine derartige Rückmeldung der zu untersuchenden Person über ihre visuelle Wahrnehmung hingegen nicht. Die vorliegende Erfindung betrifft Verfahren zur subjektiven Refraktionsbestimmung.

Herkömmlicherweise erfolgt die subjektive Refraktionsbestimmung mittels Messbrille und Messgläsern oder manuellem oder digitalem Phoropter und unter Verwendung von Sehzeichen, welche auf einem externen Monitor dargestellt werden. Phoropter sind beispielsweise unter https://de.wikipedia.org/wiki/Phoropter, Stand 13.10.2016, beschrieben. Dabei betrachtet eine zu untersuchende Person die Sehzeichen, und ein Augenoptiker oder Augenarzt setzt in die Messbrille verschiedene Messgläser mit verschiedenen Korrektionsstärken ein, oder verändert eine Korrektionseinstellung bei Verwendung eines Phoropters. Die zu untersuchende Person gibt dann eine Rückmeldung, mit welchen Messgläsern bzw. welchen Einstellungen des Phoropters die Sehzeichen am besten erkennbar sind.

Die dabei verwendeten Messgläser bzw. eine entsprechende Korrektionseinstellungen des Phoropters sind dabei jeweils einem bestimmten Refraktionsfehler zugeordnet, welcher durch das jeweilige Messglas bzw. die Einstellung des Phoropters korrigiert wird. Die vorstehend erläuterte subjektive Refraktionsbestimmung kann dabei getrennt für jedes Auge (monokular) oder auch für beide Augen gemeinsam (binokular) erfolgen.

Die oben beschriebene Herangehensweise ist im Wesentlichen ortsfest, das heißt sie ist an die Räumlichkeiten des Augenoptikers oder Augenarztes gebunden, und benötigt einen entsprechend geschulten Augenoptiker oder Augenarzt, um die Refraktionsbestimmung durchzuführen. Da aber teilweise eine Unterversorgung mit Augenoptikern oder Augenärzten vorliegt, kann es dazu kommen, dass nicht genügend Infrastruktur, das heißt nicht genügend Augenoptiker oder Augenärzte mit entsprechender Ausrüstung, vorhanden sind, um flächendeckend Refraktionsfehler zu messen und gegebenenfalls adäquat zu korrigieren, beispielsweise durch die Verschreibung von Brillen.

Bisherige mobile Lösungen, welche sich teilweise objektiver Refraktionsmethoden wie der exzentrischen Photorefraktion bedienen, weisen verschiedene Nachteile auf, da entweder relative teure spezielle Vorrichtungen und/oder geschultes Personal benötigt werden.

Zudem wird eine vereinfachte Herangehensweise angewendet, um über breite Bevölkerungsschichten eine ungefähre Messung des Refraktionsfehlers durchzuführen. Hierfür werden einfache einstellbare Brillengläser verwendet. Entsprechende Studien sind beispielsweise in Mingzhi Zhang et al., BMJ 2011, August 2011, oder in Ilechie AA. et al., Optom. Vis. Sci. 2015, April, veröffentlicht.

Bei den dort beschriebenen Verfahren wird jedoch lediglich das sogenannte sphärische Äquivalent des Refraktionsfehlers bestimmt, das heißt ein einziger Parameter. Dies ermöglicht zwar eine gewisse Korrektion von Sehfehlern und/oder statistische Untersuchungen, ist jedoch ungenauer als eine Bestimmung der drei getrennten Parameter für sphärische Brechkraft, zylindrische Brechkraft und Achslage. Andererseits werden diese Verfahren von den zu untersuchenden Personen selbst durchgeführt und benötigen kein geschultes Personal und nur relativ einfache technische Mittel, was vergleichsweise flächendeckende Untersuchungen ermöglicht.

Aus US 2006/0170864 A1 ist eine Vorrichtung gemäß dem Oberbegriff des Anspruchs 6 und ein Verfahren gemäß dem Oberbegriff des Anspruchs 12 zum Testen der Fehlsichtigkeit eines Patienten bekannt, welches eine variable Linse und Mittel zum Steuern des Brechverhaltens, insbesondere der Vorzugsrichtung der Brechkraft, der variablen Linse umfasst. Mittels der in US 2006/0170864 A1 beschriebenen Vorrichtung ist es möglich, die sphärozylindrische Refraktion des Auges durch Ausprobieren in einem System mit vielen Freiheitsgraden, abhängig vom Geschick der die Messung durchführenden Person, zu ermitteln. Durch die manuelle Steuerungseinheit kann die Winkelposition der zylindrischen Achse eingestellt werden. Zusätzlich zu den oben beschriebenen Eigenschaften lehrt US 2006/0170864 A1 nicht, wie eine Vorrichtung vorteilhaft ausgestaltet sein kann, um eine zielgerichtete Ermittlung der sphärozylindrischen Refraktion des Auges zu unterstützen.

Aus DE 10 2013 000 295 A1 sind ein Verfahren und eine Vorrichtung zur Stimulation der Akkommodation zumindest eines Auges eines Probanden bekannt, welche eine Möglichkeit zur Bestimmung eines Satzes ophthalmologischer Daten zumindest eines Auges des Probanden und insbesondere zur Messung der Fehlsichtigkeit des Probanden und zur Ermittlung einer entsprechenden optischen Korrektur ermöglicht und insbesondere eine Vorrichtung zur objektiven Refraktionsbestimmung umfasst.

Die US 2003/053027 A1 offenbart ein Computerprogramm gemäß dem Oberbegriff von Anspruch 13, eine Vorrichtung gemäß des Oberbegriffs von Anspruch 1 und ein Verfahren gemäß des Oberbegriffs von Anspruch 10.

Die DE 41 31 799 A1 offenbart Verfahren, Testzeichen und Vorrichtung zur Durchführung einer rechnergesteuerten subjektiven Refraktionsbestimmung. Verfahrensgemäß gibt ein Rechner der zu untersuchenden Person eine Aufgabe, ein Testbild und Entscheidungsalternativen vor, die vorzugsweise richtungsorientiert sind. Mittels eines Steuerelements wählt die zu untersuchende Person eine Entscheidungsalternative aus, und der Rechner entscheidet, welche Gläser, Filter und/oder dergleichen dem Auge vorgelegt oder welche Verstellungen vorgenommen werden sollen, wobei diese Schritte soweit wiederholt werden, bis für die zu untersuchende Person optische Symmetrie erreicht ist.

Aus WO 2016/045866 A1 ist eine Anzeigevorrichtung zur Demonstration optischer Eigenschaften von Brillengläsern bekannt. Eine Haltevorrichtung hält eine erstes optisches System zur Refraktionsbestimmung, welches ausgelegt ist, die subjektive Refraktion des ersten Auges zu bestimmen, wenn die Haltevorrichtung auf den Kopf des Nutzers platziert wird und/oder das erste optische Abbildungssystem ist ausgelegt, veränderbar zu sein.

Die in US 2003/0053027 A1 offenbarte Vorgehensweise ist kann für Personen mit einem starken Zylinder problematisch sein. Dies trifft ebenfalls auf die in US 2006/0170864 A1 offenbarte Möglichkeit zu.

Ausgehend von US 2003/0053027 A1 ist es daher eine erste Aufgabe der vorliegenden Erfindung, eine verbesserte Möglichkeit in Form von Vorrichtung, Computerprogramm und Verfahren bereitzustellen, um die sphärozylindrische Refraktion des Auges für Personen mit einem hohen Zylinder zuverlässig zu ermitteln.

Ausgehend von US 2006/0170864 A1 ist es daher eine zweite Aufgabe der vorliegenden Erfindung, eine verbesserte Möglichkeit in Form von Vorrichtung, Computerprogramm und Verfahren bereitzustellen, um die sphärozylindrische Refraktion des Auges für Personen mit einem hohen Zylinder zuverlässig zu ermitteln.

Hierzu werden in einem ersten Erfindungsaspekt ein System nach Anspruch 1, ein Verfahren nach Anspruch 10 und ein Computerprogramm nach Anspruch 13 bereitgestellt. Unteransprüche des ersten Erfindungsaspekts definieren weitere Ausführungsbeispiele.

In einem zweiten Erfindungsaspekt wird hierzu ein System nach Anspruch 6, ein Verfahren nach Anspruch 12 und ein Computerprogramm nach Anspruch 13 bereitgestellt. Unteransprüche des zweiten Erfindungsaspekts definieren weitere Ausführungsbeispiele.

In dem ersten Erfindungsaspekt wird ein System bereitgestellt, umfassend:

- eine Optik mit einer einstellbaren Brechkraft,
- eine Brechkrafteinstelleinrichtung zum Einstellen der Brechkraft der Optik entsprechend einem Einstellwert,
- ein Computerprogramm mit einem Programmcode, welcher, wenn er auf einer Recheneinrichtung ausgeführt wird, bewirkt, dass durch die Optik betrachtbare Sehzeichen auf einer Anzeige dargestellt werden, und

dass auf Basis von Einstellwerten der Brechkrafteinstelleinrichtung bei verschiedenen Orientierungen der Vorzugsrichtung der Sehzeichen,

ein erster Wert, der eine sphärische Brechkraft angibt, ein zweiter Wert, der eine zylindrische Brechkraft angibt, und ein dritter Wert, der eine Achslage der zylindrischen Brechkraft angibt, berechnet werden,

wobei die Berechnung basierend auf zwei Einstellwerten bei zwei verschiedenen Orientierungen der Vorzugsrichtung der Sehzeichen erfolgt,

dadurch gekennzeichnet, dass

die Berechnung zusätzlich basierend auf einer Information über eine Achslage eines Auges erfolgt, wobei die Information über die Achslage die Achslage der zylindrischen Brechkraft des Auges angibt.

Zudem wird in dem ersten Erfindungsaspekt ein Verfahren zum Ermitteln der sphärozylindrischen Refraktion eines Auges eines Benutzers bereitgestellt, umfassend:

- Darstellen von Sehzeichen bei verschiedenen Orientierungen einer Vorzugsrichtung (52) der Sehzeichen,
- Ermitteln von jeweiligen Einstellwerten einer Optik mit verstellbarer Brechkraft bei den verschiedenen Orientierungen der Vorzugsrichtung der Sehzeichen,
- Berechnen eines ersten Werts, der eine sphärische Brechkraft angibt, eines zweiten Werts, der eine zylindrische Brechkraft angibt, und eines dritten Werts, der eine Achslage der zylindrischen Brechkraft angibt basierend auf Einstellwerten bei verschiedenen Orientierungen der Vorzugsrichtung,

dadurch gekennzeichnet, dass

das Berechnen auf Basis von zwei Einstellwerten bei genau zwei verschiedenen Orientierungen der Sehzeichen und einer Information über eine Achslage eines Auges erfolgt, wobei die Information über die Achslage die Achslage der zylindrischen Brechkraft des Auges angibt.

Des Weiteren wird in dem ersten Erfindungsaspekt ein Computerprogramm mit einem Programmcode bereitgestellt, der, wenn er auf einem Prozessor ausgeführt wird, bewirkt, dass das Verfahren nach dem Verfahren des ersten Erfindungsaspekts ausgeführt wird.

In dem zweiten Erfindungsaspekt wird ein System bereitgestellt, umfassend:

- eine Optik mit einer einstellbaren Brechkraft, die eine Vorzugsrichtung der Brechkraft aufweist,
- eine Brechkrafteinstelleinrichtung zum Einstellen der Brechkraft der Optik entsprechend einem Einstellwert,
- eine Orientierungseinstelleinrichtung zum Einstellen einer Orientierung der Vorzugsrichtung der Brechkraft,

wobei das System eingerichtet ist, um einen ersten Wert, der eine sphärische Brechkraft angibt, einen zweiten Wert, der eine zylindrische Brechkraft angibt, und einen dritten Wert, der eine Achslage der zylindrischen Brechkraft angibt, auf Basis von Einstellwerten der Brechkrafteinstelleinrichtung und Einstellungen der Orientierung der Vorzugsrichtung der Brechkraft zu bestimmen,
dadurch gekennzeichnet, dass

das System ein Computerprogramm mit einem Programmcode umfasst, welcher, wenn er auf einer Recheneinrichtung ausgeführt wird, bewirkt, dass der erste Wert, der zweite Wert und der dritte Wert auf Basis von zwei Einstellwerten der Brechkrafteinstelleinrichtung bei zwei verschiedenen Orientierungen der Vorzugsrichtung der Brechkraft und basierend auf einer Information über eine Achslage eines Auges berechnet werden, wobei die Information über die Achslage die Achslage der zylindrischen Brechkraft des Auges angibt.

Zudem wird in dem zweiten Erfindungsaspekt ein Verfahren zum Ermitteln der sphärozylindrischen Refraktion eines Auges eines Benutzers bereitgestellt, umfassend:

- Ermitteln von mindestens zwei Einstellwerten einer Brechkraft einer Optik mit einer Vorzugsrichtung der Brechkraft bei verschiedenen Orientierungen der Vorzugsrichtung der Brechkraft, und
- Berechnen eines ersten Werts, der eine sphärische Brechkraft angibt, eines zweiten Werts, der eine zylindrische Brechkraft angibt, und eines dritten Werts, der eine Achslage der zylindrischen Brechkraft angibt basierend auf den mindestens zwei Einstellwerten, dadurch gekennzeichnet, dass

das Berechnen auf Basis von zwei Einstellwerten bei zwei verschiedenen Orientierungen der Vorzugsrichtung der Brechkraft erfolgt,
das Berechnen zusätzlich auf Basis von einer Information über eine Achslage eines Auges erfolgt, wobei die Information über die Achslage die Achslage der zylindrischen Brechkraft des Auges angibt.

Des Weiteren wird in dem zweiten Erfindungsaspekt ein Computerprogramm mit einem Programmcode bereitgestellt, der, wenn er auf einem Prozessor ausgeführt wird, bewirkt, dass das Verfahren nach dem Verfahren des zweiten Erfindungsaspekts ausgeführt wird.

Bei bestimmungsgemäßem Gebrauch des Systems durch einen Benutzer geben der erste Wert, der zweite Wert und der dritte Wert im Rahmen der Messgenauigkeit des Systems die sphärozylindrische Refraktion (Sphäre, Zylinder und Achse) eines Auges des Benutzers an. So kann mit relativ einfachen Mitteln einem Benutzer eine Möglichkeit zur eigenständigen Bestimmung der sphärozylindrischen Refraktion seines Auges oder seiner Augen an die Hand gegeben werden. Bestimmungsgemäßer Gebrauch bedeutet dabei insbesondere, dass der Benutzer das System entsprechend einer Anleitung benutzt. Der bestimmungsgemäße Gebrauch beinhaltet dabei, dass der Benutzer mit einem zu untersuchenden Auge durch die Optik auf Sehzeichen oder andere Elemente wie beispielsweise Gegenstände blickt und mittels der Brechkrafteinstelleinrichtung einen Einstellwert für die Brechkraft der Optik einstellt, bei der der schärfste Seheindruck vorliegt.

Bei einem bevorzugten Ausführungsbeispiel kann hierzu der Programmcode, wenn er auf der Recheneinrichtung ausgeführt wird, bewirken, dass die Recheneinrichtung Anweisungen entsprechend dem bestimmungsgemäßen Gebrauch an den Benutzer ausgibt. Dies erleichtert die Bedienung des Systems für den Benutzer.

Unter Sehzeichen sind Zeichen zu verstehen, welche die zu untersuchende Person durch die Optik hindurch betrachten kann. Verschiedene Sehzeichen können verwendet werden, beispielsweise standardisierte Optotypen (Landoltringe, Snellen-Haken, ggfs. mit daran angeschlossenen weiteren Sehzeichen wie zum Beispiel Buchstaben oder Zahlen), natürliche Bilder, Sinusgitter mit unterschiedlichen Ortsfrequenzen bei gleichen oder variierenden Kontrast, Zahlen, Buchstaben oder Symbole. Typischerweise sind dabei Sehzeichen in verschiedenen Größen dargestellt.

Bei einem bevorzugten Ausführungsbeispiel bewirkt der Programmcode, wenn er auf der Recheneinrichtung ausgeführt wird, dass durch die Optik betrachtbare Sehzeichen auf einer Anzeige dargestellt werden. Auf diese Weise werden die Sehzeichen nicht als separate Komponente benötigt. Sie können aber auch als separate Komponente, z.B. in gedruckter Form, bereitgestellt sein. Beim bestimmungsgemäßen Gebrauch der Vorrichtung betrachtet dann der Benutzer die Sehzeichen mit dem zu untersuchenden Auge durch die Optik und stellt den Einstellwert so ein, dass er die Sehzeichen möglichst scharf sieht.

Unter der Brechkraft ist, wie in der Optik üblich, der Kehrwert der Brennweite zu verstehen. Die Brechkraft wird üblicherweise in Dioptrien als gesetzliche Einheit der Brechkraft optischer Systeme in den Staaten der EU verwendet.

Eine Recheneinrichtung ist allgemein eine Einrichtung, welche (z.B. später näher erläuterte) Berechnungen durchführen kann, um aus den mindestens zwei Einstellwerten den ersten, zweiten und dritten Wert zu berechnen. Eine derartige Recheneinrichtung umfasst typischerweise zumindest einen Mikroprozessor und einen Speicher, in dem ein entsprechendes Programm abgelegt ist. Als Recheneinrichtung kann beispielsweise ein Computer, ein Smartphone oder ein Tablet-PC verwendet werden. Indem ein Computerprogramm bereitgestellt wird, kann ein Benutzer insbesondere eine eigene Recheneinrichtung (z.B. seinen/ihren eigenen Computer) verwenden. Bei anderen Ausführungsbeispielen kann das System auch die Recheneinrichtung umfassen.

Gemäß dem zweiten Erfindungsaspekt weist die Optik eine Vorzugsrichtung der Brechkraft auf, und das System umfasst weiter eine Orientierungseinstelleinrichtung zum Einstellen einer Orientierung der Vorzugsrichtung der Brechkraft der Optik. Eine Vorzugsrichtung weist die Brechkraft der Optik dann auf, wenn die Brechkraft der Optik nicht rotationssymmetrisch ist, insbesondere die Brechkraft der Optik einen von Null verschiedenen zylindrischen Anteil aufweist. Dies kann insbesondere durch eine nicht rotationssymmetrische Ausgestaltung der Optik erreicht werden.

Bei bestimmungsgemäßen Gebrauch gemäß dem zweiten Erfindungsaspekt werden die mindestens zwei Einstellwerte dann bei verschiedenen Orientierungen der Vorzugsrichtung der Brechkraft der Optik eingestellt. Auf diese Weise wird das zu untersuchende Auge entlang verschiedener Richtungen vermessen. Durch Kombination dieser Messungen können dann der erste, zweite und dritte Wert berechnet werden.

Die Orientierungseinstelleinrichtung kann beispielsweise einen Rahmen umfassen, in dem die Optik drehbar gelagert ist. Durch manuelle oder motorgetriebene Drehung der Optik können dann die verschiedenen Orientierungen eingestellt werden. Dabei kann die Orientierungseinstelleinrichtung Markierungen und/oder Rastpositionen für die Optik aufweisen, um dem Benutzer die Einstellung der Orientierungen zu erleichtern.

Bevorzugt umfasst die Orientierungseinstelleinrichtung einen Lagesensor zum Messen der Orientierung der Vorzugsrichtung der Brechkraft. Derartige Lagesensoren werden z.B. auch in Smartphones eingebaut und sind kommerziell erhältlich. Auf diese Weise kann die Orientierung automatisch erfasst und dann der Recheneinrichtung übermittelt werden. Auf diese Weise kann die tatsächliche Orientierung der Vorzugsrichtung der Brechkraft von der Recheneinrichtung bei dem Berechnen des ersten, zweiten und dritten Wertes berücksichtigt werden, so dass die Berechnung unabhängig von einer Einstellgenauigkeit der Orientierung durch den Benutzer wird.

Die Vorzugsrichtung der Brechkraft der Optik kann durch eine Achslage einer zylindrischen Brechkraft der Optik definiert sein, wobei die Brechkrafteinstelleinrichtung eingerichtet sein kann ist, entsprechend dem Einstellwert eine sphärische Brechkraft der Optik einzustellen. Eine derartige Optik mit einer festen zylindrischen Brechkraft und einer einstellbaren sphärischen Brechkraft ist relativ kostengünstig realisierbar, da nur die sphärische Brechkraft verändert werden muss.

Bei einem solchen Ausführungsbeispiel kann der Programmcode, wenn er auf der Recheneinrichtung ausgeführt wird, bewirken, dass die Recheneinrichtung, wenn die Einstellwerte bei den verschiedenen Orientierungen der Vorzugsrichtung der Brechkraft gleich sind, eine Information an den Benutzer ausgibt, dass die Brechkraft seines Auges keine messbare zylindrische Brechkraft aufweist. In diesem Fall kann der Benutzer zudem aufgefordert werden, zur Bestimmung der sphärischen Brechkraft seines Auges, eine Einstellung der Brechkraft der Optik nochmals bei veränderter zylindrischen Brechkraft der Optik durchzuführen und/oder die sphärische Brechkraft seines Auges mittels einer weiteren Optik, die keine zylindrischen Brechkraft aber eine einstellbare sphärische Brechkraft aufweist, zu bestimmen.

In einem solchen Fall, in dem das Auge keine zylindrische Brechkraft aufweist, ist mit der Herangehensweise, die Optik mit fester zylindrischer Brechkraft und einstellbarer sphärischer Brechkraft mit verschiedenen Orientierungen der Vorzugsrichtung zu verwenden, keine genaue Bestimmung der sphärischen Brechkraft des untersuchten Auges möglich. Durch die obigen zusätzliche Einstellung oder Bestimmung, zu der der Benutzer in diesem Fall aufgefordert wird, kann die sphärische Brechkraft des Auges dann bestimmt werden.

Die zylindrische Brechkraft der Optik kann zwischen 0,25 Dioptrien und 0,5 Dioptrien liegen. Bei einer zylindrischen Brechkraft unter ca. 0,25 Dioptrien ist der zylindrische Effekt durch das zu untersuchende Auge praktisch nicht wahrnehmbar, was zu fehlerhaften Messungen führen kann. Bei einer zylindrischen Brechkraft von mehr als 0,25 Dioptrien ist die Rotationssymmetrie der Brechkraft ausreichend gebrochen und die Optik weist somit eine Vorzugsrichtung auf, wodurch die oben beschriebenen Messungen mit verschiedenen Orientierungen der Vorzugsrichtung ermöglicht werden. Wird die zylindrische Brechkraft der Optik unter ca. 0,5 Dioptrien gehalten, kann er in der Berechnung des ersten, zweiten und dritten Wertes vernachlässigt werden, was die Berechnung vereinfacht.

Entsprechend dem ersten Erfindungsaspekt weisen die Sehzeichen eine Vorzugsrichtung, also keine Rotationssymmetrie, auf, wobei der Programmcode, wenn er auf der Recheneinrichtung ausgeführt wird, bewirkt, dass die Sehzeichen zeitlich nacheinander mit verschiedenen Orientierungen der Vorzugsrichtung dargestellt werden. Sehzeichen weisen insbesondere dann eine Vorzugsrichtung auf, wenn sie nicht rotationssymmetrisch sind. Bei üblichen Sehzeichen, bei denen Zeichen in Zeilen dargestellt sind, wobei die Sehzeichen von Zeile zu Zeile kleiner werden, entspricht die Zeilenrichtung der Vorzugsrichtung. Bei Landoldtringen als Sehzeichen wird die Vorzugsrichtung durch die Aussparungen der ansonsten rotationssymmetrischen Ringe definiert. In anderen Worten wird hier durch die Aussparung die ansonsten vorhandene Rotationssymmetrie gebrochen.

Die Verwendung von Sehzeichen, bei denen die Orientierung der Vorzugsrichtung verändert wird, stellt eine alternative Herangehensweise zur Verwendung einer Optik mit einer Vorzugsrichtung der Brechkraft, deren Orientierung veränderbar ist, dar. Auch durch Ändern der Orientierung der Sehzeichen kann das Auge in verschiedenen Richtungen vermessen werden, und durch Kombination der Messungen kann der erste, zweite und dritte Wert bestimmt werden.

Entsprechend den obigen Erläuterungen kann der Programmcode derart eingerichtet ist, dass, wenn er auf der Recheneinrichtung ausgeführt wird, die Recheneinrichtung den ersten Wert, den zweiten Wert und den dritten Wert basierend auf den mindestens zwei Einstellwerten bei verschiedenen Orientierungen der Vorzugsrichtung der Brechkraft und/oder bei verschiedenen Orientierungen der Vorzugsrichtung der Sehzeichen berechnet. Dass der Programmcode derart eingerichtet ist, bedeutet in diesem Zusammenhang, dass bei bestimmungsgemäßem Gebrauch, d.h. wenn der Benutzer die Einstellwerte bei den verschiedenen Orientierungen der Vorzugsrichtung der Brechkraft und/oder bei verschiedenen Orientierungen der Vorzugsrichtung der Sehzeichen korrekt bestimmt, die mittels der Programmcodes auf der Recheneinrichtung durchgeführte Berechnung des ersten, zweiten und dritten Werts im Rahmen der Messgenauigkeit des Systems Sphäre, Zylinder und Achse des untersuchten Auges ergibt.

Bei einer nicht beanspruchten Variante umfassen die mindestens zwei Einstellwerte bei verschiedenen Orientierungen der Vorzugsrichtung der Brechkraft und/oder bei verschiedenen Orientierungen der Vorzugsrichtung der Sehzeichen drei Einstellwerte bei drei verschiedenen Orientierungen der Vorzugsrichtung der Brechkraft und/oder bei drei verschiedenen Orientierungen der Vorzugsrichtung der Sehzeichen, d.h. der Programmcode ist für eine entsprechende Berechnung ausgelegt. So wird bei bestimmungsgemäßem Gebrauch der Vorrichtung das Auge in drei Richtungen vermessen, und aus den entsprechenden drei Einstellwerten können der erste, zweite und dritte Wert berechnet werden. Bevorzugt weisen die drei verschiedenen Orientierungen der Vorzugsrichtung der Brechkraft und/oder die drei verschiedenen Orientierungen der Vorzugsrichtung der Sehzeichen einen Winkelabstand von etwa 60°, z.B. zwischen 55° und 65°, zueinander auf, was die Berechnung vereinfacht.. Bei anderen Ausführungsbeispielen können auch andere Winkelabstände zwischen den Orientierungen verwendet werden, insbesondere auch Winkel kleiner als 60°.

Die Berechnung des ersten, zweiten und dritten Wertes erfolgt dabei in für sich genommen bekannter Weise. Genaueres zu diesen Berechnungen lässt sich beispielsweise in Gekeler F, Schaeffel F, Howland HC, Wattam-Bell J. Measurement of astigmatism by automated infrared photoretinoscopy. Optom. Vis Sci [Internet]. 1997; 74:472-82, verfügbar unter: http://www.ncbi.nlm.nih.gov/pubmed/9293513, entnehmen.

Alternativ können die mindestens zwei Einstellwerte bei verschiedenen Orientierungen der Vorzugsrichtung der Brechkraft, zum Beispiel im zweiten Erfindungsaspekt und/oder bei verschiedenen Orientierungen der Vorzugsrichtung der Sehzeichen, z. B. im ersten Erfindungsaspekt, zwei Einstellwerte bei zwei verschiedenen Orientierungen der Vorzugsrichtung der Brechkraft und/oder der Vorzugsrichtung der Sehzeichen umfassen, wobei der Programmcode derart eingerichtet ist, dass, wenn er auf der Recheneinrichtung ausgeführt wird, die Recheneinrichtung den ersten Wert, den zweiten Wert und den dritten Wert basierend auf den zwei Einstellwerten und basierend auf einer Information über eine Achslage und/oder einer Information über die zwei verschiedenen Orientierungen der Vorzugsrichtung der Brechkraft und/oder die zwei verschiedenen Orientierungen der Vorzugsrichtung der Sehzeichen berechnet. In einem derartigen Fall wird bei bestimmungsgemäßen Gebrauch die Achslage des untersuchten Auges ermittelt und die Information über die Achslage der Achse des Auges bereitgestellt, welche dann dem dritten Wert entspricht, sodass dann nur noch Einstellwerte für zwei verschiedenen Orientierungen zur Berechnung des ersten und zweiten Wertes benötigt werden. Diese Herangehensweise weist für Personen mit hohem Astigmatismus eine höhere Genauigkeit auf als die Herangehensweise mit drei verschiedenen Orientierungen der Vorzugsrichtung der Brechkraft und/oder mit drei verschiedenen Orientierungen der Vorzugsrichtung der Sehzeichen.

Hierzu kann der Programmcode, wenn er auf der Recheneinrichtung ausgeführt wird, bewirken, dass eine durch die Optik betrachtbare Strahlenfigur auf einer Anzeige dargestellt wird. Bei bestimmungsgemäßem Gebrauch kann der Benutzer dann mit Hilfe der Strahlenfigur die Achslage des untersuchten Auges ermitteln, indem er feststellt, welche Richtung der Strahlenfigur am schärfsten erscheint. Auf diese Weise kann dann die Information über die Achslage bereitgestellt werden.

Die zwei verschiedenen Orientierungen der Vorzugsrichtung der Brechkraft und/oder der Sehzeichen entsprechen dabei bevorzugt der Achslage und einer Richtung etwa senkrecht hierzu (z.B. in einem Winkel zwischen 85° und 95°), was einer Messung in zwei Hauptschnitten des Auges entspricht. Dies vereinfacht die Berechnung des ersten und zweiten Wertes. Die Hauptschnitte sind dabei die Achsen des Auges, die eine maximale bzw. minimale Brechkraft aufweisen. In diesem Fall stellt die Information über die Achslage gleichzeitig eine Information über die zwei verschiedenen Orientierungen der Vorzugsrichtung der Brechkraft und/oder der Sehzeichen dar.

Zu bemerken ist, dass in nicht beanspruchten Beispielen auch mehr als zwei oder drei Orientierungen der Vorzugsrichtung verwendet werden können. In diesem Fall werden mehr Einstellwerte erhalten, als zur Bestimmung des ersten, zweiten und dritten Wertes benötigt werden. Dies kann mit Hilfe herkömmlicher Verfahren der Fehlerrechnung verwendet werden, um statistische Messfehler, die durch ungenaue Einstellung der Einstellwerte hervorgerufen werden, zu verringern.

Die einstellbare Optik kann bei einem bevorzugten Ausführungsbeispiel eine Alvarezlinse umfassen. Eine Alvarezlinse umfasst dabei zwei Linsenelemente, welche gegeneinander in einer Richtung verschiebbar sind, um die Brechkraft der Alvarezlinse zu verändern. Eine Beschreibung einer derartigen Alvarezlinse findet sich beispielsweise auf http://www.spektrum.de/lexikon/optik/alvarez-linse/130, Stand 26.08.2016. So kann insbesondere die sphärische Brechkraft der Alvarezlinse in durch Verschiebung der Linsenelemente zueinander verändert werden. Zudem weisen kostengünstig erhältliche Alvarezlinsen zumeist eine zylindrische Brechkraft auf und besitzen somit eine Vorzugsrichtung. Bei einer Alvarezlinse kann die Brechkrafteinstelleinrichtung eine Stellschraube umfassen. Auf diese Weise ist die Optik mit Brechkrafteinstelleinrichtung kostengünstig implementierbar.

Bei einem anderen Ausführungsbeispiel umfasst die einstellbare Optik eine optisch veränderbare Flüssiglinse. Hierbei kann beispielsweise eine Flüssigkristallschicht zwischen Glasplatten ähnlich einer Flüssigkristallanzeige bereitgestellt sein, bei welchen Moleküle der Flüssigkristallschicht durch eine angelegte elektrische Spannung anders angeordnet werden können, beispielsweise dass sie sich von einem Mittelpunkt radial nach außen hin stärker konzentrieren. Somit kann bei derartigen Flüssigkristallanordnungen durch Anlegen einer Spannung die Brechkraft verändert werden. Derartige Flüssiglinsen sind beispielsweise in https://de.wikipedia.org/wiki/Fl%C3%BCssiglinse beschrieben. Insbesondere bei der Verwendung von Sehzeichen mit veränderbarer Orientierung der Vorzugsrichtung kann die Brechkraft einer derartigen Flüssiglinse radialsymmetrisch ohne Vorzugsrichtung sein.

Bevorzugt umfasst die Brechkrafteinstelleinrichtung eine Schnittstelle zum Übertragen der Einstellwerte an die Recheneinrichtung. So muss der Benutzer die Einstellwerte nicht manuell übertragen. Die Schnittstelle kann dabei eine drahtgebundene Schnittstelle, beispielsweise eine USB-Schnittstelle, oder eine drahtlose Schnittstelle wie eine Bluetooth-Schnittstelle oder eine WiFi-Schnittstelle sein.

Das System kann dabei als am Kopf tragbare Vorrichtung ausgestaltet sein, wobei die am Kopf tragbare Vorrichtung die Recheneinrichtung oder eine Aufnahme für die Recheneinrichtung umfasst. Damit wird dem Benutzer ein kompaktes Gerät bereitgestellt, und durch die Tragbarkeit am Kopf kann eine korrekte Positionierung am Kopf sichergestellt sein. Wenn die Aufnahme bereitgestellt ist, kann die Recheneinrichtung insbesondere ein Smartphone oder Tablet sein.

Die am Kopf tragbare Vorrichtung kann beispielsweise die Form ähnlich einer Datenbrille oder einer Virtual-Reality-Brille aufweisen. Bei einer Datenbrille kann zusätzlich zu dargestellten Sehzeichen auch die Umgebung visuell wahrgenommen werden, während bei einer Virtual-Reality-Brille eine visuelle Wahrnehmung nur über eine jeweilige Anzeige erfolgt. Die Datenbrille oder Virtual-Reality-Brille kann auch weitere Funktionen für den Benutzer bereitstellen, beispielsweise Perimetriemessungen wie in der deutschen Patentanmeldung DE 10 2014 113 682 beschrieben.

Die am Kopf tragbare Vorrichtung kann auch die Form eines Brillengestells aufweisen, in das die Optik verbaut ist. Auch so kann eine korrekte Positionierung der Optik gewährleistet werden.

Die Erfindung wird nachfolgend unter Bezugnahme auf die beigefügten Zeichnungen anhand bevorzugter Ausführungsbeispiele näher erläutert. Es zeigen:

Fig. 1 ein Blockdiagramm einer Vorrichtung gemäß einem Ausführungsbeispiel,

Figuren 2A und 2B Darstellungen einer einstellbaren Optik gemäß einem Ausführungsbeispiel,

Fig. 3 eine schematische Darstellung einer am Kopf tragbaren Vorrichtung gemäß einem Ausführungsbeispiel,

Fig. 4 ein Flussdiagramm eines Verfahrens gemäß einem Ausführungsbeispiel gemäß dem zweiten Erfindungsaspekts,

Fig. 5 eine Darstellung zur Erläuterung des Verfahrens der Figur 4,

Fig. 6 ein Flussdiagramm eines Verfahrens gemäß einem weiteren Ausführungsbeispiel,

Fig. 7 eine Darstellung zur Veranschaulichung des Verfahrens der Fig. 6,

Fig. 8 ein Flussdiagramm eines Verfahrens gemäß einem Ausführungsbeispiel gemäß dem ersten Erfindungsaspekt, und

Fig. 9 ein Strahlenkranz zur Verwendung bei dem Verfahren der Fig. 8 gemäß dem ersten Erfindungsaspekt.

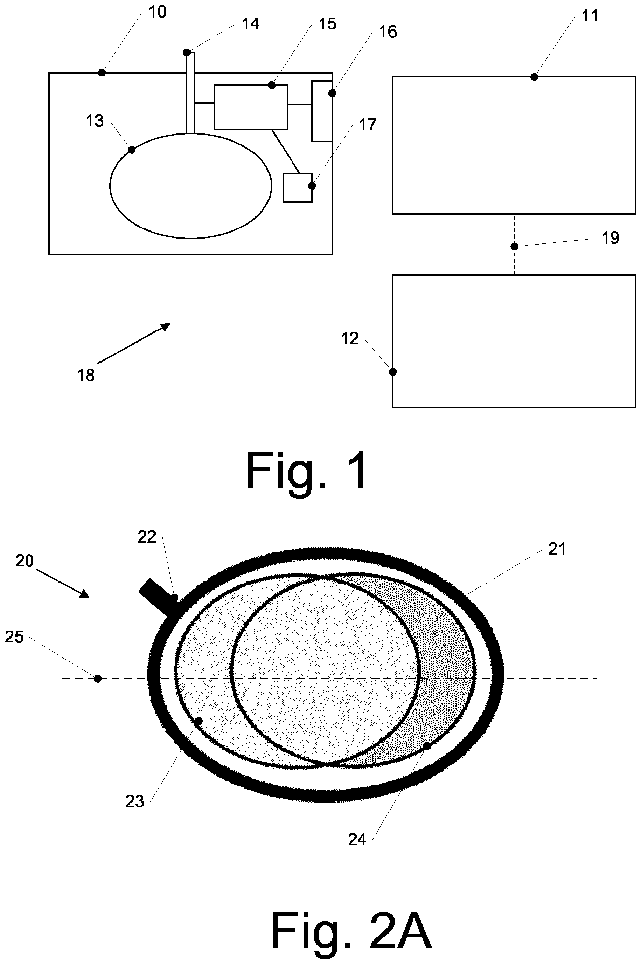

In Fig. 1 ist schematisch ein Blockdiagramm eines Systems 18 gemäß einem Ausführungsbeispiel dargestellt. Das System 18 der Fig. 1 umfasst eine Komponente 10, eine Recheneinrichtung 11 sowie eine Einrichtung 12 zur Darstellung von Sehzeichen.

Die Komponente 10 weist eine Optik 13 auf, welche mittels einer Brechkrafteinstelleinrichtung 14 hinsichtlich ihrer Brechkraft einstellbar ist. Ist die Optik 13 in der Komponente 10 beweglich, zum Beispiel drehbar, umfasst die Komponente 10 einen Lagesensor 17 zum Bestimmen einer Orientierung der Optik 13.

Die Einrichtung 12 zur Darstellung von Sehzeichen kann eine herkömmliche Sehzeichentafel sein, auf die Sehzeichen (Buchstaben, Zahlen und/oder Symbole) in verschiedenen Größen aufgedruckt sind. Alternativ kann die Einrichtung 12 ein Display (Anzeige) umfassen, welches von der Recheneinrichtung 11 wie in Figur 1 durch eine gestrichelte Linie 19 angedeutet angesteuert wird, um Sehzeichen darzustellen. Bei bestimmungsgemäßem Gebrauch sieht ein Benutzer mit einem zu untersuchenden Auge durch die Optik 13 auf die Sehzeichen der Einrichtung 12 und stellt die Optik 13 mit der Brechkrafteinstelleinrichtung 14 auf eine Brechkraft ein, mit der er die Sehzeichen am schärfsten sieht. Der entsprechende Einstellwert der Brechkrafteinstelleinrichtung 14 wird bei dem Ausführungsbeispiel der Fig. 1 in einem Speicher 15 gespeichert und dann über eine Schnittstelle 16 zu der Recheneinrichtung 11 übertragen.

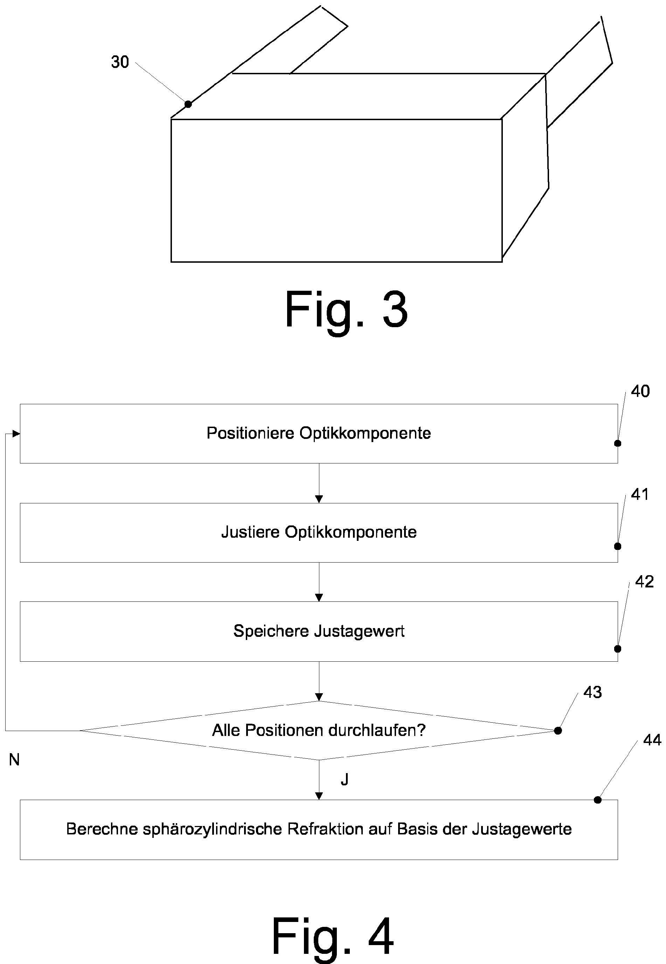

Die Recheneinrichtung 11 empfängt über die Schnittstelle 16 in dem Speicher 15 abgespeicherte Einstellwerte berechnet hieraus die Refraktion des zu untersuchenden Auges als sphärozylindrische Refraktion (Sphäre, Zylinder und Achse), also als Werte für die sphärische und zylindrische Brechkraft und die Achslage. Verschiedene Herangehensweisen hierfür werden unter Bezugnahme auf die Figuren 4-9 detaillierter erläutert.

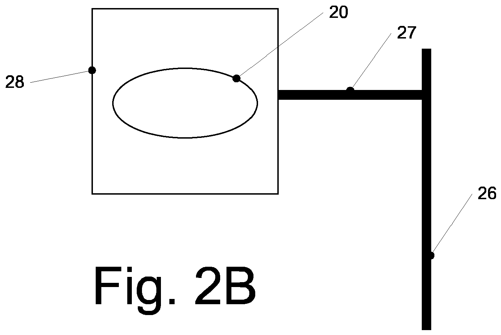

Die Figuren 2A und 2B zeigen Details möglicher Implementierungen der Optik 13. Die Figur 2A zeigt ein Beispiel für eine Optik mit einstellbarer Brechkraft eine Alvarezlinse 20. Die Alvarezlinse 20 der Fig. 2A umfasst hierzu insbesondere ein erstes Linsenelement 23 und ein zweites Linsenelement 24, die in einem Kunststoffrahmen 21 angeordnet sind. Das erste Linsenelement 23 und das zweite Linsenelement 24 sind durch Drehen einer Verstellschraube 22, die ein Beispiel für eine Brechkrafteinstelleinrichtung darstellt, entlang einer Achse 25 zueinander bewegbar, um so die sphärische Brechkraft der Alvarezlinse 20 zu verändern. Die Alvarezlinse 20 weist dabei eine feste zylindrische Brechkraft auf, welcher z.B. durch herstellungsbedingte Toleranzen erzeugt wird und typischerweise zwischen 0,25 Dioptrien und 0,5 Dioptrin liegt. Daher ist die Brechkraft der Alvarezlinse nicht rotationssymmetrisch. Die Alvarezlinse 20 weist somit eine Vorzugsrichtung auf. Für die Folgenden Erläuterungen wird angenommen, dass die Vorzugsrichtung der Achse 25 entspricht.

Wie in der Fig. 2B dargestellt, ist bei einem Ausführungsbeispiel die Alvarezlinse 20 in einem Rahmen 28 drehbar angeordnet, wobei der Rahmen 28 über ein Gestänge 26, 27 an einem festen Gegenstand, beispielsweise einem Tisch, befestigbar ist. Durch die drehbare Anordnung der Alvarezlinse 20 in dem Rahmen 28 kann eine Richtung der Achse 25 und somit eine Orientierung der Vorzugsrichtung der Alvarezlinse 20 genau eingestellt werden. An dem Rahmen 28 ist hierfür bevorzugt eine Winkelskala vorhanden, mittels der die Achse 25 auf einen bestimmten Winkel einstellbar ist. Der Rahmen 28 bietet bevorzugt zudem Rastpositionen im Abstand von 60° oder 90°, um Messungen wie nachfolgend beschrieben durchführen zu können.

Bei einem bestimmten Ausführungsbeispiel können die unter Bezugnahme auf die Fig. 1 und 2 diskutierten Komponenten, das heißt das System 18, in einer am Kopf tragbaren Vorrichtung 30 in der Art einer Virtual-Reality-Brille wie in Fig. 3 schematisch dargestellt angeordnet sein. Hierdurch wird eine kompakte Anordnung zur Refraktionsbestimmung bereitgestellt, und eine korrekte Positionierung des Systems relativ zu den Augen des Benutzers ist vereinfacht. Insbesondere kann bei einer derartigen am Kopf tragbaren Vorrichtung 30 das Element 12 als Anzeigen für beide Augen implementiert, und für beide Augen ist eine Komponente 10 bereitgestellt. Die Recheneinrichtung 11 kann ebenso in der am Kopf tragbaren Vorrichtung 30 integriert sein. Alternativ kann die Recheneinrichtung 11 und das Element 12 auch in Form eines Smartphones oder Tablets realisiert sein, wobei das Element 12 dann durch die Anzeige des Smartphones oder Tablets gebildet wird. Das Smartphone oder Tablet wird dann in eine Halterung in der am Kopf tragbaren Vorrichtung 30 eingesetzt.

Als nächstes werden unter Bezugnahme auf die Figuren 4-9 verschiedene Herangehensweisen zur Bestimmung der sphärozylindrischen Refraktion mittels der unter Bezugnahme auf die

Figuren 1-3 erläuterten Vorrichtungen dargestellt. Die im Folgenden beschriebenen Verfahren können insbesondere mit Hilfe eines Computerprogramms, welches auf der Recheneinrichtung 11 läuft, implementiert sein.

In Fig. 4 ist hierzu ein Flussdiagramm zur Veranschaulichung eines ersten Verfahrens gemäß dem zweiten Erfindungsaspekt zur Bestimmung der sphärozylindrischen Refraktion eines Auges dargestellt.

Hierzu wird in Schritt 40 eine Optik mit Vorzugsrichtung, beispielsweise die Alvarezlinse 20 der Fig. 2, mit einer ersten Orientierung der Vorzugsrichtung positioniert, und durch die Optik werden Sehzeichen betrachtet. Der Abstand der Optik zu dem zu untersuchenden Auge beträgt dabei bevorzugt 12 mm, was dem optimalen Hornhautscheitelabstand entspricht. Dieser Abstand kann durch eine Implementierung als am Kopf tragbare Vorrichtung wie in Fig. 3 sichergestellt werden oder von dem Benutzer entsprechend gewählt werden. Der Hornhautscheitelabstand beschreibt dabei den Abstand zwischen der Vorderfläche der Hornhaut des Auges und der Rückfläche der optischen Fläche der Optik. Standardmäßig wird der Hornhautscheitelabstand bei der subjektiven Refraktion als Abstand zu der Optik auf ca. 12mm angenommen bzw. eingestellt, da dies im Mittel den häufigsten Abstand bei einer getragenen Korrektionsbrille darstellt. Werden kürzere oder größere Hornhautscheitelabstände verwendet, müssen die dann für ein zu fertigendes Brillenglas verwendeten Werte der sphärozylindrischen Refraktion bzgl. der verschiedenen Hornhautscheitelabstände korrigiert werden. Diese Korrektur kann gemäß S2=S1(1+(e2-e1)S1) erfolgen. Dabei ist S1 ein bei einem ersten Hornhautscheitelabstand ermittelter erster Scheitelbrechwert, woraus ein zweiter Scheitelbrechwert S2 bei einem zweiten Hornhautscheitelabstand e2 berechnet wird. Beim Scheitelbrechwert handelt es sich um den Kehrwert der Schnittweite, also des Abstands des Brennpunktes vom Scheitel eines Brillenglases. So kann ein bei einem Hornhautscheitelabstand ermittelter Scheitelbrechwert auf einen Hornhautscheitelabstand bei einem anderen Scheitelbrechwert umgerechnet werden. Dabei wird üblicherweise nur die sphärische Brechkraft korrigiert, eine Korrektur der zylindrischen Brechkraft und deren Achslage findet üblicherweise nicht statt. In diesem Fall entspricht dann S1 der sphärischen Brechkraft bei dem Hornhautscheitelabstand e1, woraus die sphärische Brechkraft S2 beim Hornhautscheitelabstand e2 berechnet werden kann.

In Schritt 41 wird die Optik mittels der Brechwerteinstelleinrichtung auf einen Einstellwert justiert, so dass die zu untersuchende Person die Sehzeichen bestmöglich erkennt (Sehschärfe 1,0 oder bevorzugt mindestens Sehschärfe 0,8). Das Einstellen der Optik in Schritt 41 wird dabei so durchgeführt, dass die Optik zunächst auf einen Einstellwert eingestellt wird, welche einer maximal positiven Brechkraft entspricht. Das Einstellen auf die maximal positive Brechkraft kann dabei automatisiert erfolgen.

Dann wird ausgehend hiervon der Einstellwert verändert, bis die kleinsten Sehzeichen, mindestens mit der Sehschärfe 0,8 in der entsprechenden Entfernung, zum ersten Mal erkannt werden. Der entsprechende Einstellwert wird bei 42 abgespeichert. Die Sehschärfe ist dabei der Kehrwert des Auflösungsvermögens in Winkelminuten. Für eine Sehschärfe von 1,0 weist das zu erkennende Detail des Sehzeichens vom Auge aus gesehen eine Größe von 1' (Winkelminute) auf. Die Sehschärfe entspricht dabei etwa dem Arkustangens des Verhältnisses aus Größe des Sehzeichens und Entfernung des Sehzeichens vom Auge. Bei Sehschärfe 0,8 weist das zu erkennende Detail des Sehzeichens eine Größe von 1,25' auf.

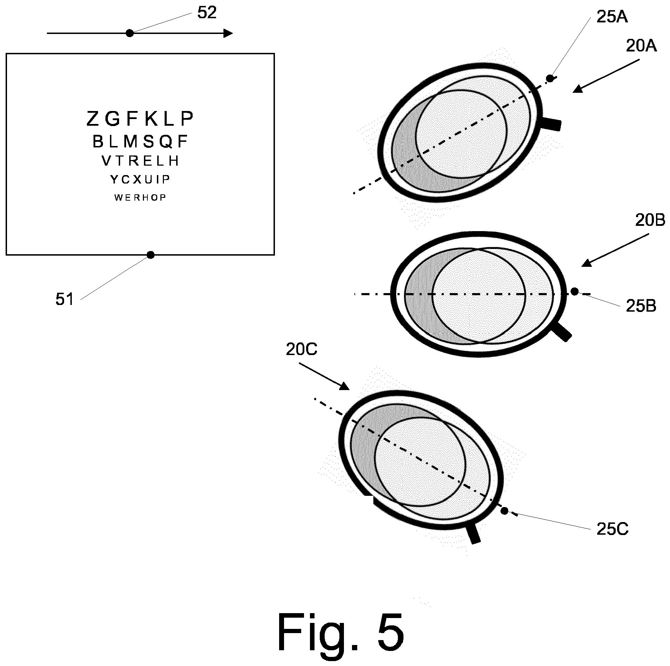

Wie durch einen Schritt 43 angedeutet, werden die Schritte 40-42 mehrmals für verschiedene Orientierungen der Vorzugsrichtung der Optik wiederholt, bis alle benötigten Positionen durchlaufen wurden. Bei dem nicht beanspruchten Beispiel der Fig. 4 werden die Schritte 40-42 insbesondere für drei verschiedene Orientierungen der Vorzugsrichtung der Optik durchlaufen. Dies ist in Fig. 5 dargestellt.

In Fig. 5 sind drei verschiedene Orientierungen für die Alvarezlinse 20 als Beispiel für die Optik gezeigt, in Fig. 5 mit 20A, 20B, 20C bezeichnet. Die jeweiligen Achsen 25, welche die Vorzugsrichtung angeben, sind mit 25A, 25B und 25C bezeichnet. Die Achsen 25A-25C stehen in einem nicht beanspruchten Beispiel in drei verschiedenen Winkeln zu einer Zeilenrichtung 52 einer Sehtafel 51. Auf der Sehtafel 51 sind Zeichen, die von Zeile zu Zeile kleiner werden, dargestellt. Die Größe der Sehzeichen unterscheidet sich dabei von Zeile zu Zeile um 0.1 logMAR Stufen, so dass die Sehzeichengröße nach dem Weber-Fechner Gesetz logarithmisch dargestellt werden. Der minimal angezeigte Sehschärfewert sollte -0.1 logMAR betragen, also einer Dezimalsehschärfe von 1.25 entsprechen. Die Sehtafel 51 ist im Falle der Fig. 5 ein einfacher bedruckter Karton. Die Achsen 25A-25C stehen dabei in Winkeln von 0° (Achse 25B), +60° (Achse 25A) und -60° (Achse 25C) zu der Zeilenrichtung 52. Allgemein werden bei dem nicht beanspruchten Beispiel der Fig. 4 in den Schritten 40-42 mindestens drei Messungen durchgeführt, wobei sich die Orientierungen der Vorzugsrichtung der Optik bei dem Ausführungsbeispiel der Fig. 5 von Messung zu Messung um etwa 60° unterscheiden.

Nachdem dies für alle Positionen der Fig. 5 durchgeführt wurde, wird in Schritt 44 die sphärozylindrische Refraktion auf Basis der bei 42 gespeicherten Einstellwerte berechnet. Dabei wird zunächst überprüft, ob für alle Orientierungen der Vorzugsrichtung der gleiche Einstellwert bei 42 gespeichert wurde. Ist dies der Fall, bedeutet dies, dass das untersuchte Auge keinen Astigmatismus aufweist, d.h. die Brechkraft des Auges keinen zylindrischen Anteil hat. In diesem Fall wird der Benutzer entsprechend informiert. Da eine genaue Bestimmung der Sphäre mit den aufgenommenen Einstellwerten nicht hinreichend genau möglich ist, kann der Benutzer dann die sphärische Brechkraft seines Auges mit einer rein sphärischen einstellbaren Linse bestimmen. Alternativ kann die zylindrische Brechkraft der Alvarezlinse 20 durch eine Verschiebung der Linsenhälften zueinander in einer Richtung senkrecht zu der Achse 25 verändert werden, und die Messung bei 42 kann in einem nicht beanspruchten Beispiel zumindest für eine Orientierung der Vorzugrichtung wiederholt werden. Aus dem so gewonnenen zusätzlichen Einstellwert sowie den schon vorliegenden Einstellwerten kann dann die sphärische Brechkraft bestimmt werden.

Auf Basis der verschiedenen Einstellwerte für die Orientierungen der Fig. 5 werden dann in einem nicht beanspruchten Beispiel Parameter A, B und D wie folgt berechnet:

$$A = \frac{Refraktion \ 0° \ (D) + Refraktion \ -60° \ (D) + Refraktion \ +60° \ (D)}{3} \qquad (1)$$

$$B = \frac{2*Refraktion\ 0°\ (D) - Refraktion\ -60°\ (D) - Refraktion\ +60°\ (D)}{3} \qquad (2)$$

$$D = \frac{Refraktion\ -60°\ (D) - Refraktion\ +60°\ (D)}{\sqrt{3}} \qquad (3)$$

Dabei bezeichnet Refraktion 0° (D) die eingestellte Brechkraft der Alvarezlinse 20B mit der Achsstellung 25B der Fig. 5 in Dioptrien, Refraktion +60° (D) die eingestellte Brechkraft der Alvarezlinse 20A mit der Achsstellung 25A der Fig. 5 in Dioptrien, und Refraktion -60° (D) die eingestellte Brechkraft der Alvarezlinse 20C bei der Achsposition 25C, d.h. jeweils den in Schritt 41 justierten und in Schritt 42 abgespeicherten Einstellwert. Für andere Winkelpositionen, welche sich nicht um jeweils 60° voneinander unterscheiden, müssen die Gleichungen (1) bis (3) in für sich genommen bekannter Weise angepasst werden. Dies ist beispielsweise in der oben erwähnten Veröffentlichung von Gekeler et al., 1997, beschrieben. Weitere Informationen zu den obigen Berechnungen können auch Thibos et al., Optometry and Vision Science Vol. 74 Nr. 6 Seiten 367-375 entnommen werden.

Aus diesen Parametern kann nun die sphärozylindrische Refraktion des untersuchten Auges wie folgt berechnet werden:

$$Sph\ddot{a}re = A + \sqrt{(B^2 + D^2)} \qquad (4)$$

$$Zylinder = -2\sqrt{(B^2 + D^2)} \qquad (5)$$

$$Achse = 0.5*\arctan\left(\frac{D}{B}\right) \qquad (6)$$

Die Werte Sphäre, Zylinder und Achse aus den Gleichungen (4) - (6) geben dann die sphärozylindrische Refraktion in Minuszylinderschreibweise an, welche in Deutschland üblicherweise bei Brillenrezepten verwendet wird. Bei der Minuszylinderschreibweise wird als sphärische Brechkraft (Sphäre) der mathematisch kleinere Hauptschnittbrechwert gewählt, wodurch der zylindrische Anteil der Brechkraft (Zylinder) ein negatives Vorzeichen erhält (siehe hierzu auch H. Goersch, Wörterbuch der Optometrie, 3. Auflage, ISBN 978-3-922269-43-4).

Wird die Messung in einem Abstand kleiner als etwa 5 m zwischen Auge und Sehtafel 51 durchgeführt (was in guter Näherung einer Unendlicheinstellung des Auges entspricht), muss die Sphäre (Gleichung 4) noch um den Betrag der Entfernung korrigiert werden. Dabei muss die Entfernung in Meter als Kehrwert in Dioptrien umgerechnet werden und die erzielte sphärische Korrektion um diesen Betrag korrigiert werden

Genaueres zu diesen Berechnungen lässt sich beispielsweise der oben erwähnten Veröffentlichung von Gekeler et al. entnehmen.

Bei der obigen Berechnung wird die zylindrisch Brechkraft der Alvarezlinse 20 vernachlässigt, was bei Werten kleiner 0,5 Dioptrien zu akzeptablen Ergebnissen führt und die Berechnung vereinfacht. Ansonsten kann die zylindrische Brechkraft der Alvarezlinse 20 auch mit herangezogen werden, wobei dann bei der Berechnung berücksichtigt werden muss, dass sich die Richtung einer gesamten Zylinderwirkung des optischen Systems aus Auge und Alvarezlinse 20 aus der Richtung und Größe der zylindrischen Brechkraft der Alvarezlinse 20 und der Achse und der zylindrischen Brechkraft des Auges bestimmt. So kann dann beispielsweise die zylindrische Brechkraft des Auges unter Berücksichtigung der sphärischen Brechkraft und zylindrischen Brechkraft der Alvarezlinse bestimmt werden.

Nach der Berechnung in Schritt 44 wird das Ergebnis dann für die untersuchende Person ausgegeben. Das Verfahren kann dann für das jeweils andere Auge wiederholt werden, um beide Augen der Person zu untersuchen.

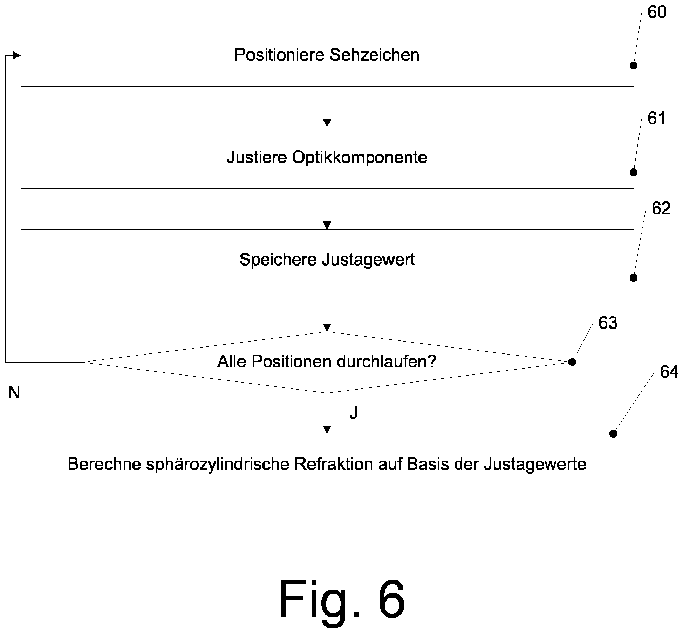

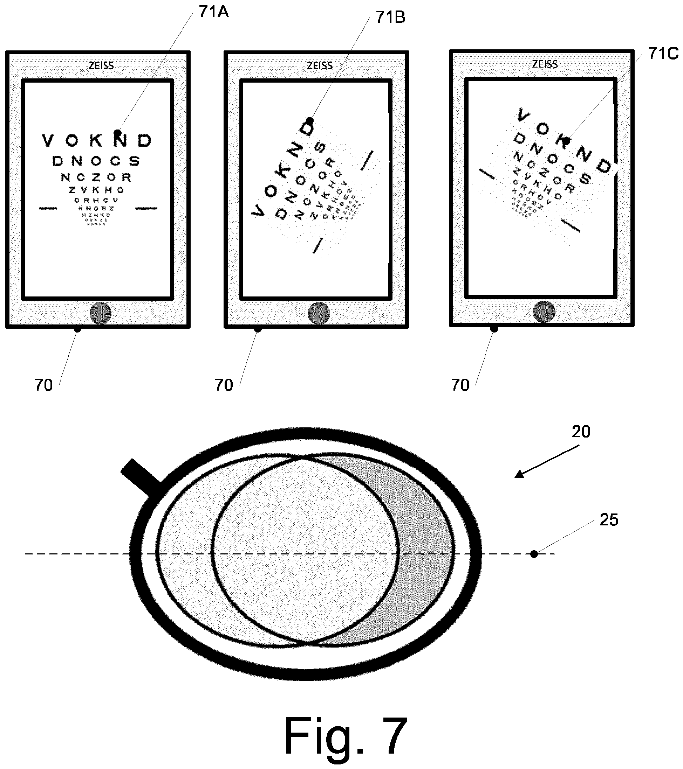

Als nächstes wird unter Bezugnahme auf die Figuren 6 und 7 eine Alternative gemäß dem ersten Erfindungsaspekt zu dem Verfahren der Figuren 4 und 5 dargestellt, bei welchem nicht die Orientierung der Vorzugsrichtung der Optik, sondern die Orientierung der Vorzugsrichtung der Sehzeichen geändert wird werden.

Die Figur 6 zeigt ein Flussdiagramm dieses Verfahrens.

In Fig. 6 werden in einem Schritt 60 Sehzeichen, bei denen eine Zeilenrichtung eine Vorzugsrichtung der Sehzeichen definiert, beispielsweise entsprechend dem ersten Erfindungsaspekt, mit einer ersten Orientierung dieser Vorzugsrichtung dargestellt. In Schritt 61 wird eine Optik dann derart eingestellt, dass die Sehzeichen möglichst gut erkennbar sind. Dies entspricht dem Einstellen des Schrittes 41 der Fig. 4 und kann wie für den Schritt 41 der Fig. 4 beschrieben erfolgen.

In Schritt 62 wird der entsprechende Einstellwert gespeichert, wie bereits für den Schritt 42 der Fig. 4 beschrieben. Die Schritte 60-62 werden wie durch einen Schritt 63 angedeutet mehrmals, in einem nicht beanspruchten Beispiel dreimal, durchlaufen, wie auch bereits für die Schritte 40-42 der Fig. 4 erläutert. Bei jedem Durchlauf werden dabei die Sehzeichen in Schritt 60 in einer anderen Winkelposition positioniert, d.h. die Orientierung der Vorzugsrichtung wird geändert. Das Verfahren der Fig. 6 unterscheidet sich also vom Verfahren der Fig. 4 dadurch, dass nicht wie bei Schritt 40 die Optik mit verschiedenen Orientierungen der Vorzugsrichtung positioniert wird, sondern in Schritt 60 die Sehzeichen in mit verschiedenen Orientierungen der Vorzugsrichtung positioniert werden. Dies wird unter Bezugnahme auf die Fig. 7 nunmehr näher erläutert.

Fig. 7 ist die Alvarezlinse 20 der Fig. 2 mit der Achse 25 dargestellt, wobei die Achse 25 in Fig. 7 in horizontaler Richtung verläuft. Auf einer Anzeige 70 werden bei drei Durchläufen des Schrittes 60 Sehzeichen mit drei verschiedenen Orientierungen der Vorzugsrichtung dargestellt, wie mit Bezugszeichen 71A, 71B und 71C in Fig. 7 bezeichnet. Die Anzeige 70 ist dabei in dem dargestellten Beispiel die Anzeige eines Tablet-Computers und ist ein Beispiel für die Implementierung der Einrichtung 12 der Fig. 1.

Bei den Sehzeichen 71A ist dabei eine Zeilenrichtung (Vorzugsrichtung) der Sehzeichen 71A parallel zu der Achse 25, was entsprechend der Darstellung der Fig. 5 als 0° Position bezeichnet wird. Die Sehzeichen 71B sind demgegenüber um +60° gedreht, die Sehzeichen 71C um -60°. Wie bei Fig. 5 werden also drei Messungen durchgeführt, wobei die Drehung zwischen den Messungen jeweils etwa 60° beträgt.

In Schritt 64 wird wie in dem Schritt 44 die sphärozylindrische Refraktion auf Basis der Einstellwerte berechnet, wobei die gleichen Formeln (1) bis (6) wie bei dem Schritt 44 zum Einsatz kommen.

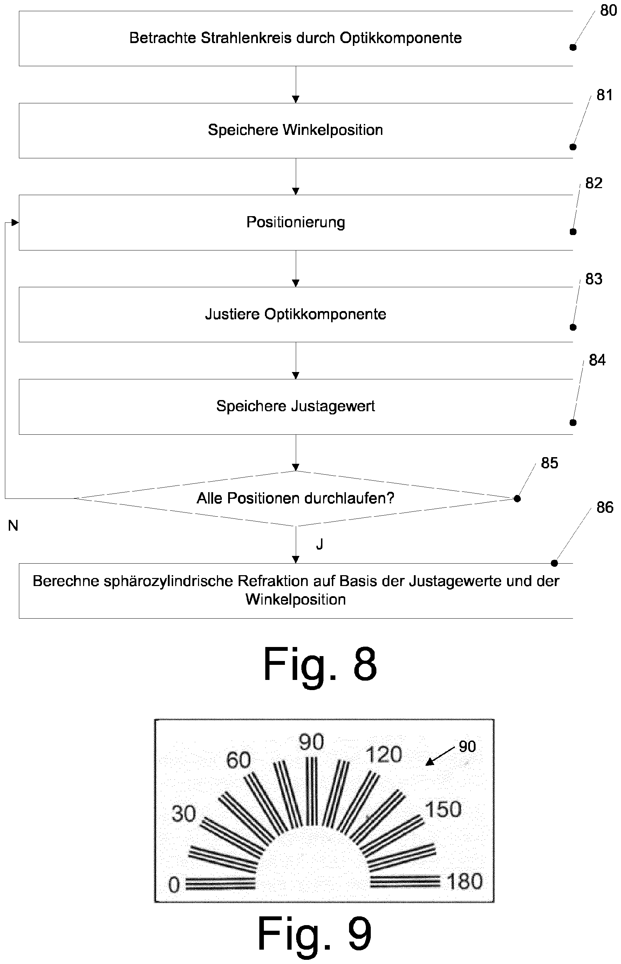

Bei den unter Bezugnahme auf die Figuren 4-7 dargestellten Ausführungsbeispielen kann es vorkommen, dass für Personen mit astigmatischen Refraktionsfehlern größer als 1,00 Dioptrien Probleme mit der korrekten Einstellung der Optik entstehen. Insbesondere für derartige zu untersuchende Personen kann ein modifiziertes Verfahren gemäß dem ersten und/oder zweiten Erfindungsaspekt durchgeführt werden, welches nunmehr unter Bezugnahme auf die Figuren 8 und 9 erläutert wird. Bei diesem Verfahren wird die Refraktionsbestimmung in den beiden sogenannten Hauptschnitten, die einen maximalen bzw. minimalen Brechwert des Auges aufweisen, durchgeführt.

Fig. 8 zeigt ein Flussdiagramm dieses Verfahrens gemäß dem ersten Erfindungsaspekt. In Schritt 80 wird auf einer Anzeige ein Strahlenkreis, insbesondere die sogenannte Strahlenfigur nach Snellen, dargestellt. Eine solche Strahlenfigur 90 ist in Fig. 9 gezeigt und weist Linien auf, die in verschiedene Richtungen zeigen. Zudem ist eine Winkelskala dargestellt.

Die Optik, beispielsweise die Alvarezlinse 20 der Fig. 2, wird dann auf die maximale positive Brechkraft eingestellt. Hierdurch wird das Auge der zu untersuchenden Person künstlich kurzsichtig gemacht. Die Strahlenfigur der Fig. 9 erscheint der Person dann an einigen Winkelpositionen sehr unscharf und an anderen Winkelposition weniger unscharf. Die untersuchende Person gibt an, an welcher Position die Strahlenfigur die beste Schärfe hat, was gleichbedeutend mit einer maximalen wahrgenommenen Schwärzung an dieser Position ist. Falls die gesamte Strahlenfigur zu unscharf ist, um dies feststellen zu können, kann die Optik auch justiert werden, bis eine derartige Unterscheidung der besten Schärfe möglich ist.

Diese Winkelposition wird dann in Schritt 81 in der Recheneinrichtung 11 der Fig. 1 gespeichert und stellt eine Information über eine Achslage des untersuchten Auges dar.

Als nächstes folgen Schritte 82 bis 85, welche den Schritten 40 bis 43 der Fig. 4 oder den Schritten 60 bis 63 der Fig. 6 entsprechen, das heißt die Optik oder die Sehzeichen werden mit verschiedenen Orientierungen ihrer Vorzugsrichtung positioniert, die Optik wird eingestellt, und der jeweilige Einstellwert wird abgespeichert. Im Unterschied zu der Fig. 4 und der Fig. 6 erfolgen die Schritte 82 bis 84 bei dem Ausführungsbeispiel gemäß dem ersten Erfindungsaspekt der Fig. 8 jedoch nur für zwei Orientierungen der Vorzugsrichtung, nämlich für eine Orientierung, bei der die Vorzugsrichtung (Achse 25 bzw. Zeilenrichtung 51) der in Schritt 80 bestimmten und Schritt 81 gespeicherte Winkelposition entspricht, und für eine Orientierung, die hierzu um 90° gedreht ist.

Wenn beispielsweise in der Winkelposition 150° der Fig. 9 die beste Schärfe erkannt wird, werden zunächst in Schritt 82 die Sehzeichen unter einem Winkel von 150° auf der Anzeige 70 der Fig. 7 dargestellt, oder die Vorzugsrichtung der einstellbaren Optik wird entsprechend der Herangehensweise der Fig. 5 unter einem Winkel von 150° ausgerichtet. Dann wird in Schritt 83 die Optik zunächst auf maximal positive Wirkung eingestellt und dann so lange justiert, bis auch die kleinsten Sehzeichen zum ersten Mal scharf erkannt werden. Dieser Einstellwert wird dann bei Schritt 84 gespeichert. Dann werden die Schritte 82 bis 84 wiederholt, wobei im Vergleich zum ersten Durchgang entweder die Sehzeichen auf der Anzeige 70 oder die Optik um ± 90° gedreht wird. Somit liegt die Brechkraftinformation für die zwei Hauptschnitte vor, aus denen die sphärozylindrische Refraktion berechnet werden kann.

In einem Zahlenbeispiel ergibt sich beispielsweise für die Position 150° eine Brechkraft von -3,0 Dioptrien und für die Position 60° (90° gedreht zu 150°) eine Brechkraft von -1,0 Dioptrien. Als sphärozylindrische Refraktion ergibt sich dann Sphäre - 1,0, Zylinder - 2,0 und Achse 60° oder Sphäre -3,0, Zylinder +2,0 und Achse 150°. Die Sphäre spiegelt hierbei den zur Achse gehörigen Brechwert dar und der Zylinder die Differenz dieses Brechwertes zu dem Brechwert, der in 90° zur Achse vorliegt.

**Patentansprüche**

1. System, umfassend:

    - eine Optik (13; 20) mit einer einstellbaren Brechkraft,
    - eine Brechkrafteinstelleinrichtung (14; 22) zum Einstellen der Brechkraft der Optik (13; 20) entsprechend einem Einstellwert,
    - ein Computerprogramm mit einem Programmcode, welcher, wenn er auf einer Recheneinrichtung (11) ausgeführt wird, bewirkt, dass durch die Optik (13; 20) betrachtbare Sehzeichen (71A-C) auf einer Anzeige (12; 70) dargestellt werden, und

    dass auf Basis von Einstellwerten der Brechkrafteinstelleinrichtung (14; 22) bei verschiedenen Orientierungen der Vorzugsrichtung (52) der Sehzeichen (71A-C) ein erster Wert, der eine sphärische Brechkraft angibt, ein zweiter Wert, der eine zylindrische Brechkraft angibt, und ein dritter Wert, der eine Achslage der zylindrischen Brechkraft angibt, berechnet werden, wobei die Berechnung basierend auf zwei der Einstellwerte bei genau zwei verschiedenen Orientierungen der Vorzugsrichtung (52) der Sehzeichen (71A-C) erfolgt, wobei die Berechnung zusätzlich basierend auf einer Information über eine Achslage eines Auges erfolgt, wobei die Information über die Achslage die Achslage der zylindrischen Brechkraft des Auges angibt.

2. System nach Anspruch 1, **dadurch gekennzeichnet, dass** die zwei Einstellwerte bei den genau zwei verschiedenen Orientierungen der Vorzugsrichtung (52) der Sehzeichen (71A-C) genau zwei Einstellwerte sind.

3. System nach Anspruch 2, wobei die genau zwei verschiedenen Orientierungen der Vorzugsrichtung (52) der Sehzeichen (71A-C) eine erste Orientierung entlang der Achslage des Auges und eine zweite Orientierung, die um 90° gegenüber der Achslage des Auges gedreht ist, umfassen.

4. System nach einem der Ansprüche 1-3, **dadurch gekennzeichnet,**
**dass** der Programmcode, wenn er auf der Recheneinrichtung (11) ausgeführt wird, bewirkt, dass eine durch die Optik (13; 20) betrachtbare Strahlenfigur (90) auf einer Anzeige (12; 70) dargestellt wird.

5. System nach Anspruch 4, **dadurch gekennzeichnet, dass** der Programmcode weiter dazu eingerichtet ist, basierend auf einer Eingabe durch einen Benutzer, welche Richtung der Strahlenfigur dem Benutzer am schärfsten erscheint, die Information über die Achslage des Auges zu bestimmen.

6. System, umfassend:

    - eine Optik (13; 20) mit einer einstellbaren Brechkraft, die eine Vorzugsrichtung (25) der Brechkraft aufweist,
    - eine Brechkrafteinstelleinrichtung (14; 22) zum Einstellen der Brechkraft der Optik (13; 20) entsprechend einem Einstellwert,
    - eine Orientierungseinstelleinrichtung (28) zum Einstellen einer Orientierung der Vorzugsrichtung (25) der Brechkraft,

    wobei das System eingerichtet ist, um einen ersten Wert, der eine sphärische Brechkraft angibt, einen zweiten Wert, der eine zylindrische Brechkraft angibt, und einen dritten Wert, der eine Achslage der zylindrischen Brechkraft angibt, auf Basis von Einstellwerten der Brechkrafteinstelleinrichtung und Einstellungen der Orientierung der Vorzugsrichtung der Brechkraft zu bestimmen, wobei das System ein Computerprogramm mit einem Programmcode umfasst, welcher, wenn er auf einer Recheneinrichtung (11) ausgeführt wird, bewirkt, dass der erste Wert, der zweite Wert und der dritte Wert auf Basis von zwei der Einstellwerte der Brechkrafteinstelleinrichtung (14; 22) bei genau zwei verschiedenen Orientierungen der Vorzugsrichtung (25) der Brechkraft und basierend auf einer Information über eine Achslage eines Auges berechnet werden, wobei die Information über die Achslage die Achslage der zylindrischen Brechkraft des Auges angibt.

7. System nach Anspruch 6, **dadurch gekennzeichnet, dass** die Orientierungseinstelleinrichtung (28) einen Lagesensor (17) zum Messen der Orientierung der Vorzugsrichtung (25) der Brechkraft umfasst.

8. System nach einem der Ansprüche 6 bis 7, **dadurch gekennzeichnet,**
**dass** die zylindrische Brechkraft der Optik (13; 20) zwischen 0,25 Dioptrien und 0,5 Dioptrien liegt.

9. Verfahren zum Ermitteln der sphärozylindrischen Refraktion eines Auges eines Benutzers, umfassend:

- Darstellen von Sehzeichen (71A-C) bei verschiedenen Orientierungen einer Vorzugsrichtung (52) der Sehzeichen (71A-C)
- Ermitteln von jeweiligen Einstellwerten einer Optik mit verstellbarer Brechkraft bei den verschiedenen Orientierungen der Vorzugsrichtung (52) der durch die Optik betrachtbaren Sehzeichen (71A-C),
- Berechnen eines ersten Werts, der eine sphärische Brechkraft angibt, eines zweiten Werts, der eine zylindrische Brechkraft angibt, und eines dritten Werts, der eine Achslage der zylindrischen Brechkraft angibt basierend auf den jeweiligen Einstellwerten bei verschiedenen Orientierungen der Vorzugsrichtung, wobei das Berechnen auf Basis von zwei der Einstellwerte bei genau zwei verschiedenen Orientierungen der Vorzugsrichtung der Sehzeichen und einer Information über eine Achslage eines Auges erfolgt, wobei die Information über die Achslage die Achslage der zylindrischen Brechkraft des Auges angibt.

10. Verfahren nach Anspruch 9, wobei die genau zwei verschiedenen Orientierungen der Vorzugsrichtung (52) der Sehzeichen (71A-C) eine erste Orientierung entlang der Achslage des Auges und eine zweite Orientierung, die um 90° gegenüber der Achslage des Auges gedreht ist, umfassen.

11. Verfahren zum Ermitteln der sphärozylindrischen Refraktion eines Auges eines Benutzers, umfassend:

- Ermitteln von mindestens zwei Einstellwerten einer Brechkraft einer Optik mit einer Vorzugsrichtung der Brechkraft bei verschiedenen Orientierungen der Vorzugsrichtung der Brechkraft, und
- Berechnen eines ersten Werts, der eine sphärische Brechkraft angibt, eines zweiten Werts, der eine zylindrische Brechkraft angibt, und eines dritten Werts, der eine Achslage der zylindrischen Brechkraft angibt basierend auf den mindestens zwei Einstellwerten, wobei das Berechnen auf Basis von zwei der Einstellwerte bei genau zwei verschiedenen Orientierungen der Vorzugsrichtung der Brechkraft erfolgt,
das Berechnen zusätzlich auf Basis von einer Information über eine Achslage eines Auges erfolgt, wobei die Information über die Achslage die Achslage der zylindrischen Brechkraft des Auges angibt.

12. Computerprogramm mit einem Programmcode, der, wenn er auf einem Prozessor ausgeführt wird, bewirkt, dass das Verfahren nach einem der Ansprüche 9-11 mittels eines entsprechenden Systems nach einem der Ansprüche 1-8 ausgeführt wird.

**Claims**

1. System, comprising:

- an optical unit (13; 20) with an adjustable refractive power,
- a refractive power setting device (14; 22) for setting the refractive power of the optical unit (13; 20) in accordance with a setting value,
- a computer program with a program code which, when executed on a computing device (11), causes optotypes (71A-C) that are observable through the optical unit (13; 20) to be displayed on a display (12; 70), and,

on the basis of setting values of the refractive power setting device (14; 22) in different orientations of the preferred direction (52) of the optotypes (71A-C),
causes a first value, which specifies a spherical refractive power, a second value, which specifies a cylindrical refractive power, and a third value, which specifies an axis position of the cylindrical refractive power, to be calculated, wherein the calculation is implemented on the basis of two of the setting values at exactly two different orientations of the preferred direction (52) of the optotypes (71A-C),
wherein the calculation is additionally implemented on the basis of an information item about an axis position of an eye, wherein the information item about the axis position specifies the axis position of the cylindrical refractive power of the eye.

2. System according to Claim 1, **characterized in that** the two setting values at the exactly two different orientations of the preferred direction (52) of the optotypes (71A-C) are precisely two setting values.

3. System according to Claim 2, wherein the precisely two different orientations of the preferred direction (52) of the optotypes (71A-C) comprise a first orientation along the axis position of the eye and a second orientation, which is rotated through 90° in relation to the axis position of the eye.

4. System according to any one of Claims 1-3, **characterized in that** the program code, when executed on the computing device (11), causes a dial (90) that is observable through the optical unit (13; 20) to be displayed on a display (12; 70).

5. System according to Claim 4, **characterized in that** the program code is further configured to determine the information item about the axis position of the eye on the basis of an input by a user as to which direction of the dial appears most sharp to said user.

6. System, comprising:

   - an optical unit (13; 20) with an adjustable refractive power, which has a preferred direction (25) of the refractive power,
   - a refractive power setting device (14; 22) for setting the refractive power of the optical unit (13; 20) in accordance with a setting value,
   - an orientation setting device (28) for setting an orientation of the preferred direction (25) of the refractive power,

   wherein the system is configured to determine a first value, which specifies a spherical refractive power, a second value, which specifies a cylindrical refractive power, and a third value, which specifies an axis position of the cylindrical refractive power, on the basis of setting values of the refractive power setting device and settings of the orientation of the preferred direction of the refractive power,
   wherein the system comprises a computer program with a program code which, when executed on a computing device (11), causes the first value, the second value and the third value to be calculated on the basis of two of the setting values of the refractive power setting device (14; 22) at exactly two different orientations of the preferred direction (25) of the refractive power and on the basis of an information item about an axis position of an eye, wherein the information item about the axis position specifies the axis position of the cylindrical refractive power of the eye.

7. System according to Claim 6, **characterized in that** the orientation setting device (28) comprises an alignment sensor (17) for measuring the orientation of the preferred direction (25) of the refractive power.

8. System according to either of Claims 6 and 7, **characterized in that** the cylindrical refractive power of the optical unit (13; 20) lies between 0.25 dpt and 0.5 dpt.

9. Method for ascertaining the spherocylindrical refraction of an eye of a user, comprising:

   - displaying optotypes (71A-C) at different orientations of a preferred direction (52) of the optotypes (71A-C),
   - ascertaining respective setting values of an optical unit with an adjustable refractive power in the different orientations of the preferred direction (52) of the optotypes (71A-C) that are observable through the optical unit,
   - calculating a first value, which specifies a spherical refractive power, a second value, which specifies a cylindrical refractive power, and a third value, which specifies an axis position of the cylindrical refractive power, on the basis of the respective setting values in different orientations of the preferred direction,

   wherein the calculation is implemented on the basis of two of the setting values at precisely two different orientations of the preferred direction of the optotypes and an information item about an axis position of an eye, wherein the information item about the axis position specifies the axis position of the cylindrical refractive power of the eye.

10. Method according to Claim 9, wherein the precisely two different orientations of the preferred direction (52) of the optotypes (71A-C) comprise a first orientation along the axis position of the eye and a second orientation, which is rotated through 90° in relation to the axis position of the eye.

11. Method for ascertaining the spherocylindrical refraction of an eye of a user, comprising:

   - ascertaining at least two setting values of a refractive power of an optical unit with a preferred direction of the refractive power in different orientations of the preferred direction of the refractive power and
   - calculating a first value, which specifies a spherical refractive power, a second value, which specifies a cylindrical refractive power, and a third value, which specifies an axis position of the cylindrical refractive power, on the basis of the at least two setting values, wherein

the calculation is implemented on the basis of two of the setting values at precisely two different orientations of the preferred direction of the refractive power,
the calculation is additionally implemented on the basis of an information item about an axis position of an eye, wherein the information item about the axis position specifies the axis position of the cylindrical refractive power of the eye.

12. Computer program with a program code which, when executed on a processor, causes the method according to any one of Claims 9-11 to be carried out by means of a corresponding system according to any one of Claims 1-8.

**Revendications**

1. Système comprenant :

- une optique (13 ; 20) ayant un pouvoir réfringent réglable,
- un dispositif de réglage du pouvoir réfringent (14 ; 22) destiné à régler le pouvoir réfringent de l'optique (13 ; 20) conformément à une valeur réglée,
- un programme informatique ayant un code de programme qui, lorsqu'il est exécuté sur un dispositif de calcul (11), a pour effet que des optotypes (71A-C) pouvant être observés à travers l'optique (13 ; 20) sont représentés sur un afficheur (12 ; 70),

sur la base de valeurs réglées du dispositif de réglage du pouvoir réfringent (14 ; 22) à différentes orientations de la direction préférentielle (52) des optotypes (71A-C),
une première valeur, qui indique un pouvoir réfringent sphérique, une deuxième valeur, qui indique un pouvoir réfringent cylindrique, et une troisième valeur, qui indique une position d'axe du pouvoir réfringent cylindrique, sont calculées,
le calcul étant effectué sur la base de deux des valeurs réglées à exactement deux orientations différentes de la direction préférentielle (52) des optotypes (71A-C),
le calcul étant effectué en plus sur la base d'une information à propos d'une position d'axe d'un œil, l'information à propos de la position d'axe indiquant la position d'axe du pouvoir réfringent cylindrique de l'œil.

2. Système selon la revendication 1, **caractérisé en ce que** les deux valeurs réglées aux exactement deux orientations différentes de la direction préférentielle (52) des optotypes (71A-C) sont exactement deux valeurs réglées.

3. Système selon la revendication 2, **caractérisé en ce que** les exactement deux orientations différentes de la direction préférentielle (52) des optotypes (71A-C) comprennent une première orientation le long de la position d'axe de l'œil et une deuxième orientation qui est tournée de 90° par rapport à la position d'axe de l'œil.

4. Système selon l'une des revendications 1 à 3, **caractérisé en ce que** le code de programme, lorsqu'il est exécuté sur le dispositif de calcul (11), a pour effet qu'une silhouette de rayons (90) pouvant être observée à travers l'optique (13 ; 20) est représentée sur un afficheur (12 ; 70).

5. Système selon la revendication 4, **caractérisé en ce que** le code de programme est en outre conçu pour déterminer l'information à propos de la position d'axe de l'œil en se basant sur une saisie par un utilisateur concernant la direction dans laquelle la silhouette de rayons apparaît la plus nette à l'utilisateur.

6. Système comprenant :

- une optique (13 ; 20) ayant un pouvoir réfringent réglable, qui possède une direction préférentielle (25) du pouvoir réfringent,
- un dispositif de réglage du pouvoir réfringent (14 ; 22) destiné à régler le pouvoir réfringent de l'optique (13 ; 20) conformément à une valeur réglée,
- un dispositif de réglage de l'orientation (28) destiné à régler une orientation de la direction préférentielle (25) du pouvoir réfringent,

le système étant conçu pour déterminer une première valeur, qui indique un pouvoir réfringent sphérique, une deuxième valeur, qui indique un pouvoir réfringent cylindrique, et une troisième valeur, qui indique une position d'axe du pouvoir réfringent cylindrique, sur la base de valeurs réglées du dispositif de réglage du pouvoir réfringent et de réglages de l'orientation de la direction préférentielle du pouvoir réfringent,

le système comportant un programme informatique comprenant un code de programme qui, lorsqu'il est exécuté sur un dispositif de calcul (11), a pour effet que la première valeur, la deuxième valeur et la troisième valeur sont calculées sur la base de deux valeurs réglées du dispositif de réglage du pouvoir réfringent (14 ; 22) à exactement deux orientations différentes de la direction préférentielle (25) du pouvoir réfringent et en se basant sur une information à propos d'une position d'axe d'un œil, l'information à propos de la position d'axe indiquant la position d'axe du pouvoir réfringent cylindrique de l'œil.

7. Système selon la revendication 6, **caractérisé en ce que** le dispositif de réglage de l'orientation (28) comporte un détecteur de position (17) destiné à mesurer l'orientation de la direction préférentielle (25) du pouvoir réfringent.

8. Système selon l'une des revendications 6 à 7, **caractérisé en ce que** le pouvoir réfringent cylindrique de l'optique (13 ; 20) est compris entre 0,25 dioptrie et 0,5 dioptrie.

9. Procédé pour identifier la réfraction sphérocylindrique d'un œil d'un utilisateur, comprenant :

- représentation d'optotypes (71A-C) à différentes orientations d'une direction préférentielle (52) des optotypes (71A-C),
- identification des valeurs réglées respectives d'une optique à pouvoir réfringent réglable aux différentes orientations de la direction préférentielle (52) des optotypes (71A-C) pouvant être observés à travers l'optique,
- calcul d'une première valeur, qui indique un pouvoir réfringent sphérique, d'une deuxième valeur, qui indique un pouvoir réfringent cylindrique, et d'une troisième valeur, qui indique une position d'axe du pouvoir réfringent cylindrique en se basant sur les valeurs réglées respectives à différentes orientations de la direction préférentielle,

le calcul étant effectué sur la base de deux des valeurs réglées à exactement deux orientations différentes de la direction préférentielle des optotypes et d'une information à propos d'une position d'axe d'un œil, l'information à propos de la position d'axe indiquant la position d'axe du pouvoir réfringent cylindrique de l'œil.

10. Procédé selon la revendication 9, les exactement deux orientations différentes de la direction préférentielle (52) des optotypes (71A-C) comprenant une première orientation le long de la position d'axe de l'œil et une deuxième orientation qui est tournée de 90° par rapport à la position d'axe de l'œil.

11. Procédé pour identifier la réfraction sphérocylindrique d'un œil d'un utilisateur, comprenant :

- identification d'au moins deux valeurs réglées d'un pouvoir réfringent d'une optique ayant une direction préférentielle du pouvoir réfringent à différentes orientations de la direction préférentielle du pouvoir réfringent, et
- calcul d'une première valeur, qui indique un pouvoir réfringent sphérique, d'une deuxième valeur, qui indique un pouvoir réfringent cylindrique, et d'une troisième valeur, qui indique une position d'axe du pouvoir réfringent cylindrique en se basant sur les au moins deux valeurs réglées,

le calcul étant effectué sur la base de deux des valeurs réglées à exactement deux orientations différentes de la direction préférentielle,

le calcul étant en outre effectué sur la base d'une information à propos d'une position d'axe d'un œil, l'information à propos de la position d'axe indiquant la position d'axe du pouvoir réfringent cylindrique de l'œil.

12. Programme informatique comprenant un code de programme qui, lorsqu'il est exécuté sur un processeur, a pour effet que le procédé selon l'une des revendications 9 à 11 est mis en œuvre au moyen d'un système correspondant selon l'une des revendications 1 à 8.

Fig. 1

Fig. 2A

Fig. 2B

# Fig. 3

Positioniere Optikkomponente — 40

Justiere Optikkomponente — 41

Speichere Justagewert — 42

Alle Positionen durchlaufen? — 43

N

J

44

Berechne sphärozylindrische Refraktion auf Basis der Justagewerte

# Fig. 4

# Fig. 5

Positioniere Sehzeichen — 60

Justiere Optikkomponente — 61

Speichere Justagewert — 62

Alle Positionen durchlaufen? — 63

N

J

Berechne sphärozylindrische Refraktion auf Basis der Justagewerte — 64

# Fig. 6

# Fig. 7

Betrachte Strahlenkreis durch Optikkomponente | 80

↓

Speichere Winkelposition | 81

↓

Positionierung | 82

↓

Justiere Optikkomponente | 83

↓

Speichere Justagewert | 84

↓

Alle Positionen durchlaufen? | 85

N

J | 86

Berechne sphärozylindrische Refraktion auf Basis der Justagewerte und der Winkelposition

# Fig. 8

# Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060170864 A1
- DE 102013000295 A1
- US 20030053027 A1
- DE 4131799 A1
- WO 2016045866 A1
- DE 102014113682

**In der Beschreibung aufgeführte Nicht-Patentliteratur**

- **MINGZHI ZHANG et al.** *BMJ 2011,* August 2011
- **ILECHIE AA et al.** *Optom. Vis. Sci.,* April 2015
- **GEKELER F ; SCHAEFFEL F ; HOWLAND HC ; WATTAM-BELL J.** Measurement of astigmatism by automated infrared photoretinoscopy. *Optom. Vis Sci,* 1997, vol. 74, 472-82, http://www.nc-bi.nlm.nih.gov/pubmed/9293513
- **THIBOS et al.** *Optometry and Vision Science,* vol. 74 (6), 367-375
- **H. GOERSCH.** Wörterbuch der Optometrie